# DEMANDE DE BREVET EUROPEEN

(11) **EP 4 556 461 A1**
(43) Date de publication de la demande: **21.05.2025**
(21) Numéro de dépôt: 24212704.1
(22) Date de dépôt: 13.11.2024
(51) Int. Cl.: C07C 253/30, C07C 255/31, C07C 303/40, C07C 311/09, H01M 10/0568

(54) **PROCÉDÉ DE SYNTHÈSE DE SELS COMPORTANT UN ANION ORGANIQUE UTILES COMME MATÉRIAUX CONDUCTEURS IONIQUES**

(30) Priorité: 16.11.2023 FR 2312567
(71) Demandeur: Commissariat à l'Energie Atomique et aux Energies Alternatives, 75015 Paris (FR)
(72) Inventeur: KUNERT, Romain, 38054 GRENOBLE CEDEX 09 (FR); BERNARD, Laurent, 38054 GRENOBLE CEDEX 09 (FR); PICARD, Lionel, 38054 GRENOBLE CEDEX 09 (FR)
(74) Mandataire: Cabinet Nony

(57) **Abrégé**

La présente invention se rapporte à un procédé pour la préparation d'un sel comportant un anion organique répondant à une formule (I) comprenant : M⁺, un cation de métal alcalin ; R¹, R², R³, R⁴, indépendamment, un radical choisi parmi : le chlore, les motifs sulfonamide anioniques de formule (IIa) : R^{a}-SO₂-N⁻-, et les motifs dinitrile anioniques de formule (IIIa) : CN-(R^{b})⁻CN, avec l'un au moins des radicaux R¹, R², R³, R⁴ étant différent du chlore ; ledit procédé comprenant au moins l'étape consistant à mettre en contact, dans un milieu solvant et en présence d'une base de métal alcalin, du chloranil avec au moins un composé de formule (II) R^{a}-SO₂-NH₂ ou (III) CN-R^{b}-CN pour former ledit sel comportant un anion organique de formule (I).

## Description

### Domaine technique

La présente invention se rapporte à des sels comportant un anion organique, en particulier utiles comme matériaux conducteurs ioniques, notamment dans des électrolytes pour batteries et vise tout particulièrement à proposer une nouvelle voie de synthèse de ces sels.

### Technique antérieure

Aujourd'hui, les batteries au lithium dominent les secteurs dans lesquels l'autonomie énergétique est un critère primordial tels que les secteurs des transports, notamment des transports électriques ou hybrides, et en particulier des véhicules individuels, de l'informatique, de la téléphonie mobile, ou de la micro-électronique. Compte-tenu de cette grande diversité d'utilisation, les attentes des batteries lithium-ion en termes de sécurité, de performances (amélioration de la conductivité ionique, de la stabilité électrochimique et du nombre de transport de l'ion lithium d'électrolytes pour batteries au lithium), et de coût sont de plus en plus exigeantes.

L'invention s'inscrit plus particulièrement au regard du développement de nouvelles générations de batteries pour l'électrisation des transports, notamment des véhicules individuels. Largement dominé par la technologie lithium-ion, le transport électrique doit envisager des nouvelles technologies pour adresser différents verrous technologiques. Les électrolytes utilisés dans les batteries au lithium sont le plus souvent liquides, et sont obtenues en dissolvant un sel très conducteur dans un solvant ou un mélange de solvants, généralement non-aqueux. Or, ces électrolytes peuvent être sujets à des emballements thermiques, et à des courts-circuits dus à l'apparition d'une croissance dendritique non maîtrisée du lithium, phénomène indésirable susceptible d'être observé au cours de la charge de la batterie.

Pour s'affranchir de ce risque d'emballement thermique, tout particulièrement indésirable pour une exploitation dans des véhicules, les électrolytes solides polymères préparés par dissolution d'un sel très conducteur dans un polymère, apparaissent comme particulièrement avantageux. Le document EP 0 850 921 A1 décrit notamment des sels conducteurs convenant à cet usage. Il s'agit de composés ioniques dérivés du malononitrile dans lesquels la charge anionique est délocalisée. Toutefois, les synthèses des composés proposés dans le document EP 0 850 921 sont notamment des synthèses requérant plusieurs étapes successives. Qui plus est, un certain nombre des précurseurs utilisés lors de ces synthèses sont relativement onéreux et/ou toxiques. Or, à la volonté d'accroissement de performance et de sécurité, s'ajoutent également les défis environnementaux, qui poussent les industriels à se tourner vers des batteries à plus faible impact environnemental, afin de s'affranchir de l'utilisation de ressources critiques.

Il demeure donc un besoin de disposer d'électrolytes notamment pour des batteries à lithium, dotés d'une stabilité électrochimique satisfaisante, d'une conductivité ionique élevée et d'un nombre de transport de l'ion lithium élevé.

Il serait en outre avantageux qu'un tel électrolyte, comprenant à titre de matériau conducteur des sels comportant un anion organique de métaux alcalins, possède une masse réduite, propice à l'obtention d'une batterie possédant une densité d'énergie augmentée, et donc une autonomie potentielle augmentée.

Il demeure également un besoin que ces électrolytes mettent à profit des sels comportant un anion organique de métaux alcalins qui soient de synthèse aisée, simplifiée et peu onéreuse. Il demeure également un besoin que ces sels comportant un anion organique de métaux alcalins pour électrolytes et leurs modes de synthèse soient respectueux à l'égard de l'environnement.

La présente invention vise précisément à répondre à ces exigences.

### Résumé de l'invention

Ainsi, l'invention concerne, selon un premier de ses aspects, un procédé pour la préparation d'un sel comportant un anion organique répondant à la formule (I) suivante : 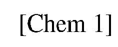 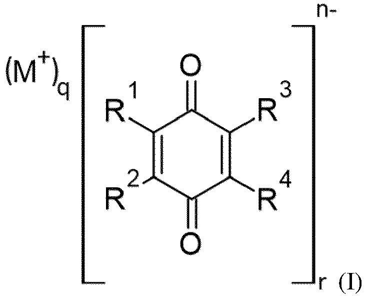 dans laquelle :
- M désigne un métal alcalin, et de préférence Li, Na ou K ;
- n représente la charge de l'anion associé et a une valeur de 1 ou 2 ;
- q et r désignent indépendamment l'un de l'autre 1 ou 2, avec la valeur de q étant ajustée pour neutraliser la charge négative du composé de formule (I) ;
- R¹, R², R³, R⁴ désignent indépendamment l'un de l'autre un radical choisi parmi :
   - un atome de chlore,
   - les motifs sulfonamide anioniques de formule (IIa) :

      R^{a}-SO₂-N⁻- (IIa)

      dans laquelle R^{a} désigne une chaîne aliphatique, linéaire ou ramifiée, saturée ou insaturée,
      comprenant de 1 à 10 atomes de carbone, de préférence 1 à 5 atomes de carbone, ladite chaîne étant éventuellement substituée par un ou plusieurs atomes de fluor, et
   - les motifs dinitrile anioniques de formule (IIIa) : 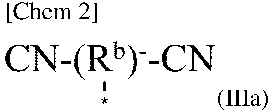
      dans laquelle * désigne la position de la liaison covalente avec le cycle et R^{b} désigne un motif hydrocarboné trivalent, linéaire ou ramifié, saturé ou insaturé, comprenant de 1 à 10 atomes de carbone, de préférence 1 à 4 atomes de carbone, plus préférentiellement 1 atome de carbone ;
      avec l'un au moins des radicaux R¹, R², R³, R⁴ étant différent d'un chlore et ledit procédé comprenant au moins l'étape consistant à mettre en contact, dans un milieu solvant et en présence d'une base de métal alcalin, du 2,3,5,6-tétrachlorocyclohexa-2,5-diène-1,4-dione (ou chloranil) avec :
         i) au moins un composé de formule (II) :
            R^{a}-SO₂-NH₂ (II), avec Ra étant tel que défini en formule (IIa), ou
         ii) au moins un composé de formule (III) :
            CN-R^{b}-CN (III), avec Rb étant tel que défini en formule (IIIa)
            pour former ledit sel comportant un anion organique de formule (I).

Comme il ressort des exemples qui suivent, les inventeurs ont constaté qu'il est possible d'obtenir des sels comportant un anion organique de formule (I) selon un procédé très simplifié puisque ne requérant qu'une unique étape réactionnelle consistant à faire réagir au moins un composé de formule (II) ou (III) avec du chloranil et ayant en outre l'avantage de considérer, à titre de matières premières, des produits disponibles commercialement, peu onéreux et à impact très réduit à l'égard de l'environnement.

Selon un mode de réalisation particulier, le sel comportant un anion organique de formule (I) tel que décrit précédemment comprend un unique radical R¹, R², R³ ou R⁴ différent d'un atome de chlore.

Selon un mode de réalisation particulier, le sel comportant un anion organique de formule (I) tel que décrit précédemment comprend deux radicaux R¹, R², R³, R⁴, de préférence identiques, différents d'un atome de chlore.

Selon un mode de réalisation, la substitution nucléophile d'au moins un composé de formule (II) ou (III) sur le chloranil est réalisée en présence d'une base de métal alcalin choisie parmi les hydroxydes de métaux alcalins et les hydrures de métaux alcalins.

Selon un autre de ses aspects, la présente invention se rapporte à l'utilisation d'un sel comportant un anion organique de formule (I) obtenu selon le procédé de l'invention à titre d'électrolyte.

Selon un autre de ses aspects, la présente invention se rapporte à un électrolyte, notamment pour systèmes électrochimiques, formé en tout ou partie d'au moins un sel comportant un anion organique de formule (I) obtenu selon le procédé de l'invention.

D'autres caractéristiques, variantes et avantages des procédés, molécules et électrolytes selon l'invention, de leur préparation et de leur mise en oeuvre ressortiront mieux à la lecture de la description, des exemples qui vont suivre, donnés à titre illustratif et non limitatif de l'invention.

Dans la suite du texte, les expressions « compris entre « ... et ... », « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

### Description détaillée de l'invention

### PROCÉDÉ DE L'INVENTION

Comme précisé ci-dessus, le procédé selon l'invention est caractérisé par l'étape de mise en contact, dans un milieu solvant et en présence d'une base de métal alcalin, du 2,3,5,6-tétrachlorocyclohexa-2,5-diène-1,4-dione (ou chloranil) avec :
i) au moins un composé de formule (II) : R^{a}-SO₂-NH₂ (II), avec R^{a} désignant une chaîne aliphatique, linéaire ou ramifiée, saturée ou insaturée, comprenant de 1 à 10 atomes de carbone, de préférence 1 à 5 atomes de carbone, ladite chaîne étant éventuellement substituée par un ou plusieurs atomes de fluor, ou
ii) au moins un composé de formule (III) : CN-R^{b}-CN (III), avec R^{b} désignant un motif hydrocarboné trivalent, linéaire ou ramifié, saturé ou insaturé, comprenant de 1 à 10 atomes de carbone, de préférence 1 à 4 atomes de carbone, plus préférentiellement 1 atome de carbone.

De façon avantageuse, le procédé de l'invention est représentatif d'une synthèse dite *one-pot* du produit de la réaction, c'est-à-dire requérant une unique étape de synthèse à partir du composé chloranil, en tant que produit de départ, pour former le sel comportant un anion organique de formule (I) attendu. Le chloranil est mis en contact avec au moins un composé de formule (II) ou (III), dans les conditions selon l'invention pour former le sel comportant un anion organique de formule (I). Le procédé selon l'invention ne requiert pas d'étape supplémentaire du type isolement voire purification de composé intermédiaire entre le produit de départ qu'est le chloranil et le sel comportant un anion organique final de formule (I). La réaction a lieu dans un unique mélange réactionnel. Bien entendu, et comme détaillé ci-après, le sel comportant un anion organique de formule (I) ainsi formé, peut faire l'objet d'opérations réactionnelles consécutives en vue de l'isoler de son milieu de synthèse voire de le purifier.

Il peut être considéré que la réaction du chloranil avec le composé de formule (II) ou (III) relève d'une ou plusieurs substitutions nucléophiles aromatiques selon le nombre de motifs de formule (IIa) ou (IIIa) attendus au niveau du motif anionique formant le sel comportant un anion organique de formule (I).

La quantité en réactif chloranil peut varier de 0,1 à 15 % en poids, de préférence de 0,5 à 12 % en poids, plus préférentiellement de 1 à 10 % en poids, mieux de 2 à 8 % en poids, par rapport au poids total du milieu réactionnel.

La quantité en composé(s) de formule (II) ou (III), peut varier de 0,1 à 15 % en poids, de préférence de 0,15 à 12 % en poids, plus préférentiellement de 0,25 à 10 % en poids, mieux de 0,5 à 5 % en poids, par rapport au poids total du milieu réactionnel.

Il est entendu que la quantité en composé de formule (II) ou (III) est ajustée au regard du niveau de substitution nucléophile désiré sur le chloranil. Cet ajustement relève des compétences de l'homme du métier. Pour une monosubstitution, on privilégiera un mélange équimolaire du chloranil et du composé de formule (II) ou (III) choisi. Pour une disubstitution, on privilégiera un mélange dans lequel le composé de formule (II) ou (III) est à raison d'au moins 2 équivalents pour 1 équivalent de chloranil.

Selon un premier mode de réalisation, le procédé selon l'invention met en contact du chloranil avec au moins un composé de formule (II) R^{a}-SO₂-NH₂, avec R^{a} tel que décrit précédemment.

Les composés de formule (II) R^{a}-SO₂-NH₂, avec R^{a} tel que décrit précédemment, sont notamment choisis parmi les (C₁-C₁₀)alkylsulfonamides, et de préférence les (C₁-C₅)alkylsulfonamides, les trifluoro(C₁-C₁₀)alkylsulfonamides et de préférence les trifluoro(C₁-C₅)alkylsulfonamides, les perfluoro(C₂-C₁₀)alkylsulfonamides, et de préférence les perfluoro(C₂-C₅)alkylsulfonamides et leurs mélanges, plus préférentiellement c'est la trifluorométhanesulfonamide.

Selon un deuxième mode de réalisation, le procédé selon l'invention met en contact du chloranil avec au moins un composé de formule (III) CN-R^{b}-CN, avec R^{b} tel que décrit précédemment.

Les composés de formule (III) CN-R^{b}-CN, avec R^{b} tel que décrit précédemment, sont notamment choisis parmi les (C₃-C₁₀)alkyldinitriles linéaires ou ramifiés, saturés ou insaturés, et leurs mélanges et en particulier parmi le propanedinitrile (ou malononitrile), le butanedinitrile (ou succinonitrile), le pentanedinitrile (ou glutaronitrile), l'hexanedinitrile (ou adiponitrile), l'heptanedinitrile (ou pimélonitrile), l'octanedinitrile (ou subéronitrile), le nonanedinitrile (ou azélanitrile), le décanedinitrile (ou sébaconitrile), et leurs mélanges, plus préférentiellement étant le malononitrile.

L'ordre de mise en contact du chloranil avec au moins un composé de formule (II) ou (III) peut varier. Ainsi, le composé de formule (II) ou (III) peut être ajouté à une suspension ou solution de chloranil, ou alors le chloranil peut être ajouté à une suspension ou solution de composé (II) ou (III). De préférence, le composé de formule (II) ou (III), notamment associé à une base telle que détaillée ci-après, est ajouté à une suspension ou solution de chloranil.

Comme précisé ci-dessus, la réaction selon l'invention est effectuée en présence d'une base de métal alcalin déterminant la nature du cation M dans la formule (I).

Cette base de métal alcalin est de préférence un hydroxyde de métal alcalin ou un hydrure de métal alcalin.

Comme hydroxydes de métaux alcalins, on peut citer l'hydroxyde de lithium LiOH, l'hydroxyde de sodium NaOH, l'hydroxyde de potassium KOH, hydratés ou non, et leurs mélanges.

Comme hydrures de métaux alcalins, on peut citer l'hydrure de lithium LiH, l'hydrure de sodium NaH, l'hydrure de potassium KH, et leurs mélanges.

La quantité en base de métal alcalin est ajustée de sorte qu'au moins 2 équivalents de base, voire plus, soient utilisés pour 1 équivalent de composé de formule (II) ou (III).

De préférence, l'hydroxyde de métal alcalin ou l'hydrure de métal alcalin seul ou en présence d'un composé de formule (II) ou (III) est ajouté au milieu réactionnel comprenant le réactif chloranil. L'hydroxyde de métal alcalin ou l'hydrure de métal alcalin peut être ajouté au milieu réactionnel en une unique fois ou en plusieurs fois en cours de synthèse.

Selon une variante de réalisation, le procédé met en oeuvre du chloranil avec de la trifluorométhanesulfonamide à titre de composé de formule (II) tel que décrit précédemment, et en présence d'hydrure de lithium.

Selon une variante de réalisation, le procédé met en oeuvre du chloranil avec du malononitrile à titre de composé de formule (III), et en présence d'un hydroxyde de métal alcalin. Comme précisé ci-dessus, l'étape de mise en contact dans le procédé est réalisée dans un milieu solvant. Le choix de ce milieu solvant relève clairement des compétences de l'homme du métier.

Comme exemples de milieux solvants convenant à l'invention, on peut citer les solvants volatils, tels que l'acétone, l'acétonitrile, le tétrahydrofurane (THF), en particulier le THF anhydre, et leurs mélanges.

Par solvant volatil, on entend un solvant possédant une pression de vapeur saturante à 20°C supérieure ou égale à 3 kPa.

On peut également citer les solvants non volatils, comme l'eau ou un mélange hydroalcoolique, par exemple un mélange éthanol-eau, ou isopropanol-eau.

De préférence le milieu solvant est choisi parmi l'acétonitrile, le tétrahydrofurane (THF), en particulier le THF anhydre, et leurs mélanges.

De préférence, le milieu réactionnel est maintenu, dans des conditions standard de pression, à une température allant de -5°C à 100°C, plus particulièrement de -5°C à 90°C, mieux de 0°C à 85°C, étant entendu que la température peut être maintenue à une certaine température au départ de la réaction, puis être augmentée. Selon un mode de réalisation particulier, la température est maintenue à 0°C au début de la réaction puis est laissée revenir à température ambiante, en particulier à environ 25°C.

La durée de la réaction dépend de la nature chimique du composé de formule (II) ou (III) impliqué, et varie entre 5h et 35h, de préférence entre 10h et 30h, plus préférentiellement entre 15h et 30h. Durant toute la durée de la réaction, le milieu réactionnel est avantageusement maintenu sous agitation.

Selon le procédé de l'invention, on obtient à l'issue de la réaction un mélange contenant le sel comportant un anion organique de formule (I) attendu, généralement en solution ou en suspension dans le milieu réactionnel.

Le procédé selon l'invention comprend avantageusement une étape de purification consécutive à la réaction permettant de récupérer le produit de la réaction exempt d'impureté(s).

Cette étape de purification peut comprendre une étape de filtration du milieu réactionnel. Selon la solubilité du produit de la réaction, le résidu de la filtration ou alors le filtrat obtenu après filtration peuvent être récupérés.

L'étape de purification peut également comprendre une étape d'ajout d'un solvant au milieu réactionnel ou au résidu ou au filtrat obtenus lors d'une étape de filtration précédente. Comme exemples de solvants utiles, on peut citer, sous forme anhydre ou non, le THF, le dichlorométhane (DCM), l'acétonitrile, l'acétone, le méthanol, et leurs mélanges. Le solvant peut être ajouté sous forme anhydre afin de limiter l'introduction d'eau dans le milieu réactionnel ou le filtrat obtenu. Lors de cette étape, le solvant est ajouté jusqu'à obtenir un milieu réactionnel ou un filtrat limpide, qui peut ensuite être concentré, en particulier concentré à l'évaporateur rotatif ou en étant placé sous vide.

L'étape de purification peut comprendre une étape de dessication, c'est-à-dire un séchage à l'étuve, sous pression réduite à l'évaporateur rotatif et/ou sous vide, ou alors séchés à l'aide d'un ou plusieurs agents desséchants, comme par exemple du pentoxyde de phosphore P₂O₅. L'ensemble de ces modes opératoires, de même que leurs conditions réactionnelles relèvent clairement des compétences de l'homme du métier.

Une fois l'étape de purification terminée, on obtient le produit de la réaction pur, qui peut être caractérisé par spectroscopie d'absorption infra-rouge à transformée de Fourier ou FTIR (de l'anglais *Fourier Transform InfraRed spectroscopy*), spectroscopie de résonance magnétique nucléaire au fluor (¹⁹F RMN) ou au carbone (¹³C RMN), ou encore spectroscopie d'ionisation électrospray en couple avec une spectrométrie de masse (ESI-MS, de l'anglais *Electrospray Ionisation* - *Mass Spectrometry*)*.*

Le procédé permet ainsi de préparer un sel comportant un anion organique de formule (I) tel que décrit précédemment.

Selon un mode de réalisation particulier, le sel comportant un anion organique de formule (I) tel que décrit précédemment comprend un unique radical R¹, R², R³ ou R⁴ différent d'un atome de chlore.

Selon une variante de ce mode de réalisation, l'unique radical R¹, R², R³, R⁴ différent d'un atome de chlore désigne un motif anionique choisi parmi les (C₁-C₅)alkylsulfonamides, les trifluoro(C₁-C₅)alkylsulfonamides, les perfluoro(C₂-C₅)alkylsulfonamides et le malononitrile.

Selon un autre mode de réalisation particulier, le sel comportant un anion organique de formule (I) tel que décrit précédemment comprend deux radicaux R¹, R², R³, R⁴, de préférence identiques, différents d'un atome de chlore.

Selon une variante de ce mode de réalisation, les sels comportant un anion organique répondent à la formule (Ia) suivante : 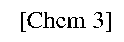 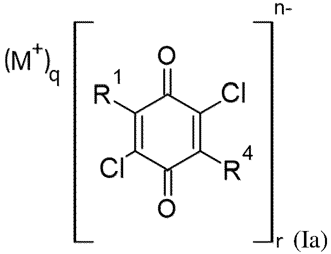 dans laquelle M, n, q et r sont tels que définis précédemment et les radicaux R¹, R⁴, sont identiques, et de préférence désignent un motif anionique choisi parmi les (C₁-C₅)alkylsulfonamides, les trifluoro(C₁-C₅)alkylsulfonamides, les perfluoro(C₂-C₅)alkylsulfonamides et le malononitrile.

À titre représentatif des sels comportant un anion organique de formule (I) et (Ia), peuvent être tout particulièrement cités les sels comportant un anion organique ci-dessous dans lesquels M⁺ désigne Li⁺, Na⁺ et/ou K⁺, et le cas échéant, y₁ et y₂ sont des nombres entiers allant de 0 à 4 :

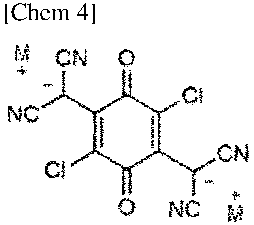

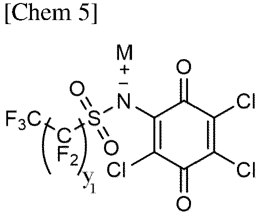

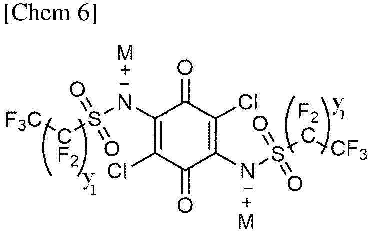

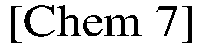

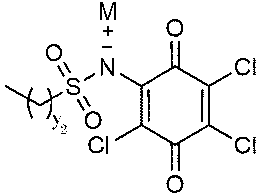

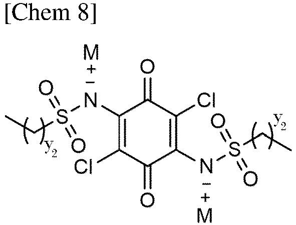

Le procédé selon l'invention s'avère ainsi tout particulièrement avantageux pour préparer les sels comportant un anion organique suivants :
- le sel de lithium du 2,3,5-trichloro-6-propanedinitrile-1,4-benzoquinone (composé A) : 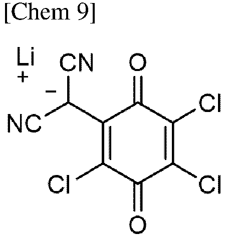
- le sel de sodium du 2,3,5-trichloro-6-propanedinitrile-1,4-benzoquinone (composé B) : 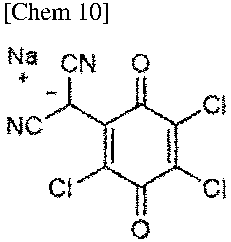
- le sel de potassium du 2,3,5-trichloro-6-propanedinitrile-1,4-benzoquinone (composé C) : 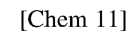 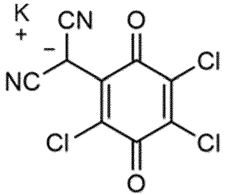
   le sel de disodium du 2,5-dichloro-3,6-di(propanedinitrile)-1,4-benzoquinone (composé D) : 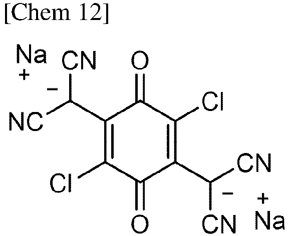
   et le sel de lithium de la 2,3,5-trichloro-6-trifluorométhanesulfonamide-1,4-benzoquinone (composé E) :

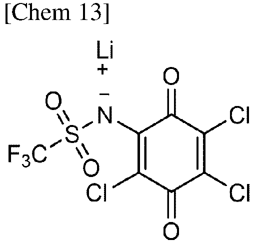

Ainsi, le procédé selon l'invention permet, via une synthèse *one-pot,* d'obtenir un produit de réaction avec un rendement allant de 60 à 75 %, possédant une pureté allant de 93 à 97%.

### UTILISATION DES COMPOSES OBTENUS SELON L'INVENTION COMME ELECTROLYTE

Selon un autre de ses aspects, la présente invention se rapporte à un électrolyte, notamment pour systèmes électrochimiques, comprenant au moins un sel comportant un anion organique de formule (I) tel que décrit précédemment, voire étant formé en tout ou partie d'un tel sel comportant un anion organique.

Les sels comportant un anion organique de formule (I) tels que décrits précédemment peuvent être avantageusement utilisés en solution dans des solvants organiques de type carbonates en tant qu'électrolytes liquides, mais également dans des électrolytes solides, dans des matrices polymères ou des électrolytes solides composites.

En particulier, les sels comportant un anion organique de formule (I) tels que décrits précédemment comprenant deux radicaux R¹, R², R³, R⁴ différents d'un atome de chlore, *a fortiori* les sels comportant un anion organique de formule (Ia) tels que décrits précédemment, peuvent connaître de multiples applications comme conducteurs ioniques dans des électrolytes.

Ces sels comportant un anion organique possèdent une masse molaire faible tout en portant une charge positive totale élevée, grâce à la présence de leurs deux contre-cations. L'utilisation de tels sels comportant un anion organique permet d'offrir des électrolytes ayant un faible poids dans une batterie complète. Ceci permet également d'offrir des batteries ayant un maximum de densité d'énergie, et donc d'autonomie potentielle. L'électrolyte selon l'invention peut être mis en oeuvre dans un système électrochimique, par exemple une batterie cation-ion, tel qu'une batterie lithium-ion, sodium-ion, ou potassium-ion.

Les sels comportant un anion organique de formule (I) selon l'invention sont donc utiles afin d'obtenir des batteries sodium-ion ou potassium-ion qui offrent une alternative aux batteries lithium-ion, de façon à s'affranchir des métaux critiques comme le lithium.

La présente invention concerne également, selon encore un autre de ses aspects, un système électrochimique comprenant un électrolyte selon l'invention.

Selon un mode de réalisation particulier, l'électrolyte est mis en oeuvre dans une batterie, en particulier une batterie au lithium, une batterie au sodium, ou une batterie au potassium.

La présente invention concerne également, selon un autre de ses aspects, l'utilisation de sels comportant un anion organique obtenus selon le procédé de l'invention comme matériaux d'électrode capables de réaction rédox.

L'invention va maintenant être décrite au moyen des exemples suivants, soumis à titre illustratif et non limitatif de l'invention.

### Exemples

### EXEMPLE 1

### Synthèse du composé A selon l'invention

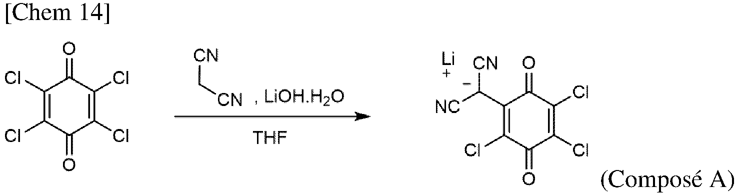

Une suspension de chloranil (1,73 g, 6,98 mmol) et de malononitrile préalablement purifié par sublimation (458 mg, 6,94 mmol) est placée sous atmosphère d'argon dans 22 mL de THF. A la suspension est ajouté de l'hydroxyde de lithium monohydraté (638 mg, 15,1 mmol) avec une spatule, sur une période de 5 minutes. La solution est ensuite agitée sous argon pendant 16h. La solution est filtrée sur Celite^{®} pour éliminer l'hydroxyde de lithium en excès. Le filtrat est évaporé et le produit brut est suspendu dans du dichlorométhane, collecté et rincé abondamment. Le produit est séché sous vide sur P₂O₅ pendant 48h, puis sous vide plus poussé (< 0.1 mbar) à 80°C pendant 48h. Le composé A est obtenu sous la forme d'une poudre bleue-noire (1,33 g, rendement = 68 %).

La pureté du produit a été confirmée par FTIR, ¹³C RMN et ESI-MS.

NMR (400 MHz, THF-d₈) ⁷Li: δ: 0.25 ppm (référence externe LiBF₄, δ : -2.59 ppm). ¹³C: δ: 176.51, 165.63, 143.93, 143.08, 136.50, 120.66, 109.61, 50.80 ppm.

ESI-MS(-) : m/z = 272.90. [C₉N₂O₂Cl₃]- : Masse théorique = 272.90.

FTIR : v = 2218, 1676, 1600, 1497, 1222, 1098, 719 cm⁻¹.

### EXEMPLE 2

### Synthèse du composé B selon l'invention

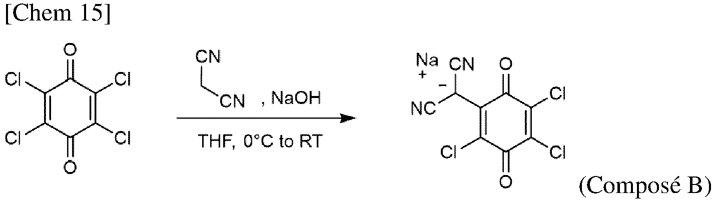

Une suspension de chloranil (1,94 g, 7,82 mmol) et de malononitrile préalablement purifié par sublimation (517 mg, 7,83 mmol) est placée sous atmosphère d'argon dans 25 mL de THF anhydre. La suspension est refroidie avec un bain de glace, puis de l'hydroxyde de sodium (682 mg, 16,7 mmol) est ajouté. La solution est ensuite agitée sous argon pendant 20h à température ambiante. La solution est filtrée sur Celite^{®}. Le filtrat est évaporé et le produit brut est suspendu dans du dichlorométhane, collecté et rincé abondamment. Le produit est séché sous vide sur P₂O₅ à 50°C, puis sous vide plus poussé (< 0.1 mbar) à 120°C pendant une nuit. Le composé B est obtenu sous la forme d'une poudre bleu sombre (2,12 g, rendement = 91 %).

La pureté du produit a été confirmée par FTIR, ¹³C RMN et ESI-MS.

¹³C NMR (400 MHz, MeOD): δ: 177.32, 167.63, 144.51, 144.32, 137.51, 121.64, 110.50, 68.84 ppm.

ESI-MS(-) : m/z = 272.91. [C₉N₂O₂Cl₃]- : Masse théorique = 272.90.

FTIR : v = 2200, 2186, 1678, 1575, 1489, 1217, 1093, 719, 605 cm⁻¹.

### EXEMPLE 3

### Synthèse du composé C selon l'invention

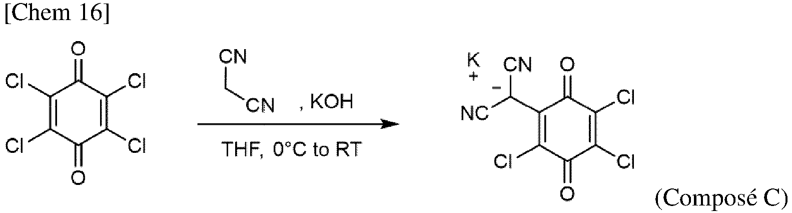

Une suspension de chloranil (1,96 g, 7,88 mmol) et de malononitrile préalablement purifié par sublimation (521 mg, 7,88 mmol) est placée sous atmosphère d'argon dans 25 mL de THF anhydre. La suspension est refroidie avec un bain de glace, puis de l'hydroxyde de potassium broyé (981 mg, 16,1 mmol) est ajouté. La solution est ensuite agitée pendant 17h à température ambiante. Le précipité obtenu est collecté et rincé abondamment avec du tetrahydrofurane puis du dichloromethane jusqu'à l'obtention d'un filtrat limpide. De l'acétone est ajoutée et la solution est filtrée sur Celite^{®}. Le filtrat est évaporé, séché sous pression réduite sur P₂O₅ à 50°C puis sous vide plus poussé (< 0.1 mbar) à 120°C pendant une nuit. Le produit est broyé sous atmosphère inerte et encore séché sous vide poussé à 120°C pendant 2 jours. Le composé C est obtenu sous la forme d'une poudre bleu sombre (1,62 g, rendement = 66 %).

La pureté du produit a été confirmée par FTIR, ¹³C RMN et ESI-MS.

¹³C NMR (400 MHz, DMSO-d₆): δ: 175.84, 165.24, 142.27, 142.09, 135.90, 120.30, 108.01, 48.90 ppm.

ESI-MS(-) : m/z = 272.90. [C₉N₂O₂Cl₃]- : Masse théorique = 272.90.

FTIR : v = 2207, 2191, 1677, 1603, 1506, 1214, 1097, 721, 601 cm⁻¹.

### EXEMPLE 4

### Synthèse du composé D selon l'invention

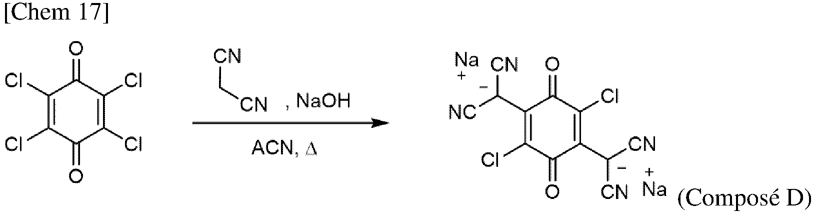

À une solution de malononitrile (780 mg, 11,8 mmol) dans 20 mL d'acétonitrile est ajouté de l'hydroxyde de sodium broyé (972 mg, 23,8 mmol). La solution est agitée pendant 15 minutes avant d'être ajoutée à une suspension de chloranil (1,94 g, 5,94 mmol) dans 15 mL d'acétonitrile. Le ballon qui contenait la solution de malononitrile est rincé plusieurs fois, de sorte que le milieu réactionnel comprend 80 mL d'acétonitrile au total. Le milieu réactionnel est chauffé à reflux pendant 28h, puis laissé revenir à température ambiante. Le milieu réactionnel est filtré et lavé avec de l'acétonitrile et de l'acétone. Un produit brut est récupéré et solubilisé dans un mélange 1:1 acétone/méthanol, et filtré sur Celite^{®}. Le filtrat est évaporé et le produit est suspendu dans du tétrahydrofurane. Le produit est collecté et rincé abondamment avec du tétrahydrofurane puis du dichlorométhane. Le produit est ensuite séché sous pression réduite sur P₂O₅ à 50°C puis sous vide plus poussé (< 0.1 mbar) à 120°C pendant une nuit. Le produit est broyé sous atmosphère inerte et encore séché sous vide poussé à 120°C pendant 2 jours. Le composé D est obtenu sous la forme d'une poudre marron (1,32 g, rendement = 64%).

La pureté du produit a été confirmée par FTIR, ¹³C RMN et ESI-MS.

¹³C NMR (400 MHz, DMSO-d₆): δ: 170.52, 145.43, 121.61, 106.19, 47.37 ppm.

ESI-MS(-) : m/z = 302.95 [C₁₂N₄O₂Cl₂]- : Masse théorique = 302.95.

FTIR : v = 2212, 2185, 1588, 1498, 1292, 608 cm⁻¹.

### EXEMPLE 5

### Synthèse du composé E selon l'invention

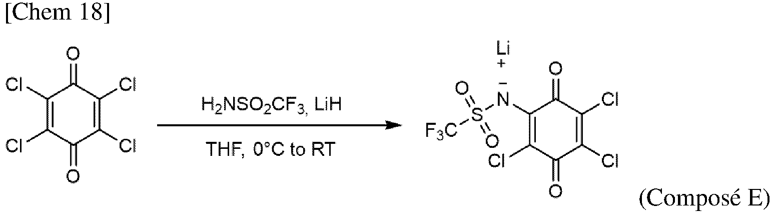

En boîte-à-gants, le trifluorométhanesulfonamide (270 mg, 1,76 mmol) est solubilisé dans 5 mL de tétrahydrofurane anhydre dans un tube de Schlenk. De l'hydrure de lithium LiH (28,0 mg, 3,52 mmol) est ajouté à l'aide d'une spatule, et la solution est laissée à agiter pendant 1h30 après la fin de dégazage d'hydrogène, menant à un précipité blanc.

Sur ligne de Schlenk, une suspension de chloranil (432 mg, 1.74 mmol) dans 10 mL de tétrahydrofurane anhydre est refroidie avec un bain de glace. Avec une canule de transfert, la suspension de trifluorométhanesulfonamide lithié (la suspension ayant été rincée plusieurs fois avec du tétrahydrofurane anhydre) y est ajoutée. Le milieu réactionnel est agité pendant 20h en laissant revenir à température ambiante. La solution est filtrée avec un papier filtre sur canule de transfert. Le filtrat est évaporé puis suspendu dans du dichlorométhane anhydre. Le produit violet sombre est lavé abondamment avec du dichlorométhane anhydre. Le produit est séché sous pression réduite sur P₂O₅ puis sous vide plus poussé (< 0.1 mbar) à 120°C pendant une nuit. Le composé E est obtenu sous la forme d'une poudre violet sombre (403 mg, rendement = 64%).

NMR (400 MHz, MeOD) ⁷Li: δ: 1.40 ppm (ref externe LiBF4: -0.39 ppm). ¹⁹F: δ: -81.57 ppm (93% de pureté avec 7% de sous-produit di-substitué à δ: -81.71 ppm, ref externe C₆F₆ : -165.38 ppm).

¹³C NMR : δ: 174.26, 173.02, 149.40, 142.26, 138.43, 122.92, 120.38 ppm.

ESI-MS(-) : m/z = 355.87 [C₇Cl₃F₃NO₄S]- : Masse théorique = 355.86. Produit di-substitué observé à m/z = 468.86.

FTIR : v = 1696, 1642, 1532, 1392, 1180, 1074 cm⁻¹.

## Revendications

1. Procédé pour la préparation d'un sel comportant un anion organique répondant à la formule (I) suivante : 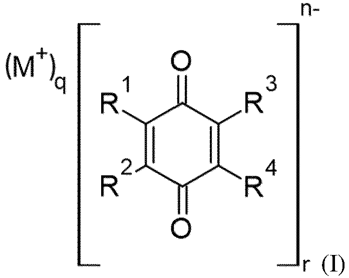 dans laquelle :
• M désigne un métal alcalin, et de préférence Li, Na ou K ;
• n représente la charge de l'anion associé et a une valeur de 1 ou 2 ;
• q et r désignent indépendamment l'un de l'autre 1 ou 2, avec la valeur de q étant ajustée pour neutraliser la charge négative du composé de formule (I) ;
• R¹, R², R³, R⁴ désignent indépendamment l'un de l'autre un radical choisi parmi :
- un atome de chlore,
- les motifs sulfonamide anioniques de formule (IIa) :
R^{a}-SO₂-N⁻- (IIa)
dans laquelle R^{a} désigne une chaîne aliphatique, linéaire ou ramifiée, saturée ou insaturée, comprenant de 1 à 10 atomes de carbone, de préférence 1 à 5 atomes de carbone, ladite chaîne étant éventuellement substituée par un ou plusieurs atomes de fluor, et
- les motifs dinitrile anioniques de formule (IIIa) : 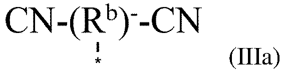
dans laquelle * désigne la position de la liaison covalente avec le cycle et R^{b} désigne un motif hydrocarboné trivalent, linéaire ou ramifié, saturé ou insaturé, comprenant de 1 à 10 atomes de carbone, de préférence 1 à 4 atomes de carbone, plus préférentiellement 1 atome de carbone ;
avec l'un au moins des radicaux R¹, R², R³, R⁴ étant différent d'un chlore et ledit procédé comprenant au moins l'étape consistant à mettre en contact, dans un milieu solvant et en présence d'une base de métal alcalin, du 2,3,5,6-tétrachlorocyclohexa-2,5-diène-1,4-dione ou chloranil avec :
i) au moins un composé de formule (II) :
R^{a}-SO₂-NH₂ (II), avec Ra étant tel que défini en formule (IIa), ou
ii) au moins un composé de formule (III) :
CN-R^{b}-CN (III), avec Rb étant tel que défini en formule (IIIa)
pour former ledit sel comportant un anion organique de formule (I).

2. Procédé selon la revendication 1, dans lequel le sel comportant un anion organique de formule (I) comprend un unique radical R¹, R², R³ ou R⁴ différent d'un atome de chlore.

3. Procédé selon la revendication 1, dans lequel le sel comportant un anion organique de formule (I) comprend deux radicaux R¹, R², R³, R⁴, de préférence identiques, différents d'un atome de chlore.

4. Procédé selon la revendication 3, dans lequel le sel comportant un anion organique répond à la formule (Ia) suivante : 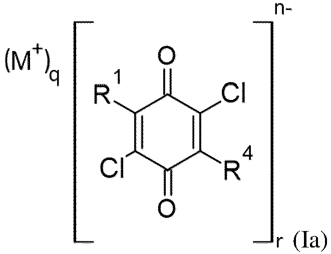 dans laquelle M, n, q et r sont tels que définis en revendication 1 et les radicaux R¹, R⁴, sont identiques.

5. Procédé selon l'une quelconque des revendications précédentes, dans lequel le sel comportant un anion organique est obtenu après l'étape de mise en contact du chloranil avec au moins un composé de formule (II) tel que défini en revendication 1, notamment choisi parmi les (C₁-C₁₀)alkylsulfonamides, et de préférence les (C₁-C₅)alkylsulfonamides, les trifluoro(C₁-C₁₀)alkylsulfonamides et de préférence les trifluoro(C₁-C₅)alkylsulfonamides, les perfluoro(C₂-C₁₀)alkylsulfonamides, et de préférence les perfluoro(C₂-C₅)alkylsulfonamides et leurs mélanges.

6. Procédé selon l'une quelconque des revendications 1 à 4, dans lequel le sel comportant un anion organique est obtenu après l'étape de mise en contact du chloranil avec au moins un composé de formule (III) tel que défini en revendication 1, notamment choisi parmi les (C₃-C₁₀)alkyldinitriles linéaires ou ramifiés, saturés ou insaturés, et leurs mélanges, en particulier parmi le propanedinitrile ou malononitrile, le butanedinitrile, le pentanedinitrile, l'hexanedinitrile, l'heptanedinitrile, l'octanedinitrile, le nonanedinitrile, le décanedinitrile, et leurs mélanges, et plus préférentiellement étant le malononitrile.

7. Procédé selon l'une quelconque des revendications précédentes, dans lequel la quantité en réactif chloranil varie de 0,1 à 15 % en poids, de préférence de 0,5 à 12 % en poids, plus préférentiellement de 1 à 10 % en poids, mieux de 2 à 8 % en poids, par rapport au poids total du milieu réactionnel.

8. Procédé selon l'une quelconque des revendications précédentes, dans lequel la quantité en composé(s) de formule (II) ou (III) varie de 0,1 à 15 % en poids, de préférence de 0,15 à 12 % en poids, plus préférentiellement de 0,25 à 10 % en poids, mieux de 0,5 à 5 % en poids, par rapport au poids total du milieu réactionnel.

9. Procédé selon l'une quelconque des revendications précédentes, dans lequel ladite base est choisie parmi les hydroxydes de métaux alcalins, comme l'hydroxyde de lithium LiOH, l'hydroxyde de sodium NaOH, l'hydroxyde de potassium KOH, hydratés ou non, et leurs mélanges, et les hydrures de métaux alcalins, comme l'hydrure de lithium LiH, l'hydrure de sodium NaH, l'hydrure de potassium KH, et leurs mélanges.

10. Procédé selon l'une quelconque des revendications précédentes, dans lequel le milieu solvant est choisi parmi l'acétonitrile, le tétrahydrofurane (THF), en particulier le THF anhydre, et leurs mélanges.

11. Procédé selon l'une quelconque des revendications précédentes, dans lequel le milieu réactionnel est maintenu à une température allant de -5°C à 100°C, plus particulièrement de -5°C à 90°C, mieux de 0°C à 85°C.

12. Procédé selon l'une quelconque des revendications précédentes, comprenant en outre une étape de purification.

13. Utilisation d'un sel comportant un anion organique de formule (I) obtenu selon le procédé décrit selon l'une quelconque des revendications 1 à 12, à titre d'électrolyte.

14. Électrolyte, notamment pour systèmes électrochimiques, formé en tout ou partie d'au moins un sel comportant un anion organique de formule (I) obtenu selon le procédé décrit selon l'une quelconque des revendications 1 à 12.

15. Système électrochimique comprenant un électrolyte selon la revendication précédente.

16. Utilisation de sels comportant un anion organique de formule (I) obtenus selon le procédé décrit selon l'une quelconque des revendications 1 à 12 comme matériaux d'électrode capables de réaction rédox.
